(19) Europäisches Patentamt
European Patent Office
Office européen des brevets

(11) Publication number: 0 017 425 A1

# (12) EUROPEAN PATENT APPLICATION

(21) Application number: 80300941.4

(22) Date of filing: 26.03.80

(51) Int. Cl.$^3$: A 61 K 31/43
C 07 D 499/74, C 07 D 499/76

(30) Priority: 02.04.79 US 26456

(43) Date of publication of application: 15.10.80 Bulletin 80/21

(84) Designated Contracting States: BE DE FR GB IT NL

(71) Applicant: PRESIDENT AND FELLOWS OF HARVARD COLLEGE
17 Quincy Street
Cambridge Massachusetts 02138(US)

(72) Inventor: Fisher, Jed Freemen
4128 12th Avenue
South Minneapolis Minnesota(US)

(72) Inventor: Knowles, Jeremy Randall
44 Coolidge Avenue
Cambridge Massachusetts(US)

(74) Representative: Perry, Robert Edward et al,
GILL JENNINGS & EVERY 53-64 Chancery Lane
London WC2A 1HN(GB)

## (54) N-Acyl-6-aminopenicillanic acid derivatives, their use as beta-lactamase deactivators, and compositions containing them and beta-lactam antibiotics.

(57) N-Acyl-6-aminopenicillanic acid sulfones, including methicillin sulfone and the novel compounds quinacillin sulfone and cloxacillin sulfone, whose corresponding N-acyl-6-aminopenicillanic acid has a rate constant not more than 100 seconds $^{-1}$, are of utility as β-lactamase deactivators and can be administered to animals being treated with a β-lactam antibiotic. Compositions comprise the sulfones, optionally in admixture with a β-lactam antibiotic and/or a pharmaceutical carrier.

EP 0 017 425 A1

Harvard
GJE 6180/59

# N-ACYL-6-AMINOPENICILLANIC ACID DERIVATIVES, THEIR USE AS β-LACTAMASE DEACTIVATORS, AND COMPOSITIONS CONTAINING THEM AND β-LACTAM ANTIBIOTICS.

This invention relates to N-acyl-6-aminopenicllanic acid derivatives, some of which are novel per se, to their use as β-lactamase deactivators, and to compositions containing them and β-lactam antibiotics.

The resistance of certain bacteria to β-lactam antibiotics such as penicillins and cephalosporins is frequently due to β-lactamases, bacerial enzymes which catalyse the rapid hydrolysis of the β-lactam ring of the antibiotics. One approach to the problem of overcoming this resistance has been to use antibiotics which, because of their structure, are poor substrates for the hydrolytic bacterial enzymes. The cephamycins disclosed by Nagarajan et al, J.A.C.S. 93 (1971) 2308-2310, are such hydrolysis-resistant antibiotics.

Another approach has been to use a β-lactamase deactivator which is itself not an effective antibiotic, in conjunction with an antibiotic, to protect the antibiotic from hydrolysis. One such deactivator is clavulanic acid, disclosed by Howarth et al, J.S. Chem. Commun. (1976) 276-277; Reading et al, Antimicrob. Agents Chemother. 11 (1977) 952-857 and Dumon et al, Antimicrob. Agents Chemother. 15 (1979) 315-317. Other compounds which deactivate bacterial hydrolytic enzymes are some carbapenam compounds such as, for example, PS-5 disclosed by Okamura et al, J. Antibiotics 31 (1978) 480-482 and the olivanic acid derivatives disclosed by Brown et al, J.C.S.

Chem. Commun. (1977) 953-954 and Maeda et al, J. Antibiotics 30 (1977) 770-773. Clavulanic acid and the carbapenams have all been isolated from natural sources.

Synthetic compounds have also been used to deactivate β-lactamases. Examples are 6β-bromo-des-aminopenicillanic acid disclosed by Pratt et al, Proc. Natl. Acad. Sci. U.S.A. 75 (1978) 4145-4149 and Knott-Hunziker et al, Biochem. J. 177 (1979) 365-367; 6-des-aminopenicillanic acid sulfone (CP-45899) of formula I

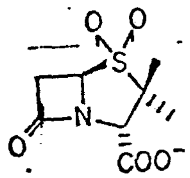

I

disclosed by English et al, Antimicrob. Agents Chemother. 14 (1978) 414-419 and Belgian Patent Specification No. 867,859; and 6-chloro-des-aminopenicillanic acid sulfone disclosed by Cartwright et al, Nature 278 (1979) 360-361.

Methicillin sulfone, of formula II

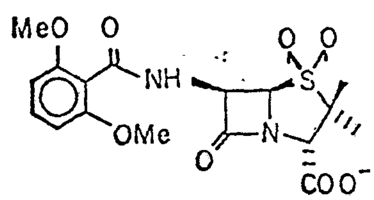

II

is disclosed by Johnson et al, J. Org. Chem. 28 (1963) 1927, but no utility is ascribed to this compound therein.

According to the present invention, N-acyl derivatives of 6-aminopenicillanic acid sulfone whose corresponding N-acyl derivative of 6-aminopenicillanic acid has a rate constant ($k_{cat}$) of not more than 100, preferably not more than 25, seconds$^{-1}$, are of utility as β-lactamase deactivators and can be used for that purpose in animals being treated with a β-lactam antibiotic.

The N-acyl-6-aminopenicillanic acid sulfones can be administered to any animal, including humans, with a bacterial infection, either alone, if the patient is being treated with a β-lactam antibiotic, or in admixture with a β-lactam antibiotic. In either case, if desired, a suitable pharmaceutically acceptable non-toxic carrier substance may be used. The relative proportions of the sulfone and the antibiotic administered to the patient or present in the mixture may be from 1:100 to 100:1 by weight, depending on specific circumstances. The dosage consequently can vary over a wide range depending upon the dosage of the desired β-lactam antibiotic, which conventionally ranges from 50 mg to 1 g per kg body weight. Administration can be oral, intravenous, intramuscular or intraperitoneal, or by any other medically recognised method. When administered with a carrier, an effective amount of the active agent should be present in the carrier to provide the desired dosage. Any carrier will usually be a solid or a sterile liquid.

It has been discovered that, after the administration of certain β-lactamase deactivators, including CP-45899 and the compounds used in the present invention, intermediates are formed which are covalently attached to the lactamases. It is believed that these acyl-enzyme intermediates either deacylate (resulting in the undesirable restoration of the lactamase) or fragment (resulting in the desirable deactivation of the lactamase). Since fragmentation occurs in competition with deacylation, the number of fragmentations, and hence the effectiveness of the deactivator, can be increased by increasing the lifespan of the acyl-enzyme intermediates. This can be achieved by employing an N-acyl derivative of 6-aminopenicillanic acid sulfone in which the corresponding N-acyl derivative of 6-aminopenicillanic acid is a very poor substrate for β-lactamase. Since the rate constant ($k_{cat}$) of an N-acyl derivative of 6-aminopenicillanic acid is a measure of its ability to act as a substrate, this constant provides the basis for defining the class of sulfones

used in the present invention.

Preferred derivatives for use in the invention are the known compound methicillin sulfone and the novel compounds quinacillin sulfone

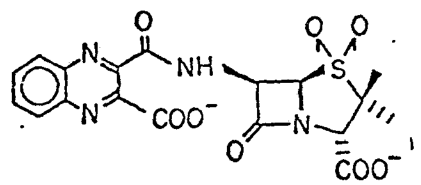

and cloxacillin sulfone

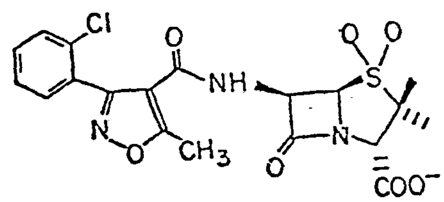

The N-acyl derivatives of 6-aminopenicillanic acid sulfone are synthesised by oxidising the appropriate N-acyl derivatives of 6-aminopenicillanic acid with aqueous permanganate, according to the procedure described by Johnson et al, J. Org. Chem. 28 (1963) 1927. For example, quinacillin sulfone is prepared by oxidising quinacillin with aqueous permanganate.

The following Table indicates the comparative efficacies of the four compounds CP-45899, methicillin sulfone, quinacillin sulfone and cloxacillin sulfone, based on the in vitro treatment of E. coli RTEM β-lactamase.

In the Table, "$k_{cat}$" is the rate constant of the corresponding N-acyl derivative of penicillanic acid; "$t_{\frac{1}{2}}$" is the time taken for the activity of the β-lactamase to be halved; "Turnovers" represents the number of turnovers before inactivation, i.e. the number of molecules of the compounds needed to inactivate one β-lactamase molecule.

| Compound | $k_{cat}$ (sec$^{-1}$) | $t_{\frac{1}{2}}$ (min) | Turnovers |
|---|---|---|---|
| CP-45899 | 40 | 44 | 4,500 |
| methicillin sulfone | 10 | ~1 | 22,500 |
| quinacillin sulfone | 7 | ~1 | 400 |
| cloxacillin sulfone | 24 | 1.5 | 17,000 |

The Table shows that quinacillin sulfone is the most effective of the four compounds and also that the corresponding N-acyl derivative of 6-aminopenicillanic acid (i.e. quinacillin itself) has the lowest $k_{cat}$ value, which indicates poor substrate capability. The effectiveness of quinacillin sulfone is shown in that both the inactivation time and the number of turnovers before inactivation are low. The Table shows that methicillin sulfone and cloxacillin sulfone are also effective deativating agents, although less effective than quinacillin sulfone.

CLAIMS

1. An N-acyl derivative of 6-aminopenicillanic acid sulfone, in which the rate constant ($k_{cat}$) of the corresponding N-acyl derivative of 6-aminopenicillanic acid is not more than 100 seconds$^{-1}$, characterised by its use as a β-lactamase deactivator.

2. An N-acyl derivative of 6-aminopenicillanic acid sulfone, in which the rate constant ($k_{cat}$) of the corresponding N-acyl derivative of 6-aminopenicillanic acid is not more than 100 seconds$^{-1}$, characterised by its use in an animal being treated with a β-lactam antibiotic.

3. An N-acyl derivative according to claim 1 or claim 2 in which the rate constant is not more than 25 seconds$^{-1}$.

4. An N-acyl derivative according to any of claims 1 to 3 which is methicillin sulfone.

5. Quinacillin sulfone.

6. Cloxacillin sulfone.

7. A pharmaceutical composition comprising a β-lactam antibiotic and a β-lactamase deactivator, characterised in that the β-lactamase deactivator is an N-acyl derivative of 6-aminopenicillanic acid sulfone according to any of claims 1 to 6.

8. A composition according to claim 7 additionally comprising a pharmaceutically acceptable, non-toxic carrier.

9. A pharmaceutical composition comprising quinacillin sulfone and a pharmaceutically acceptable, non-toxic carrier.

10. A pharmaceutical composition comprising cloxacillin sulfone and a pharmaceutically acceptable, non-toxic carrier.

11. A pharmaceutical composition comprising a β-lactamase deactivator and a pharmaceutically acceptable, non-toxic carrier which is a solid or a sterile liquid, characterised in that the β-lactamase deactivator is methicillin sulfone.

0017425

European Patent Office

Application number EP 80 30 0941

# EUROPEAN SEARCH REPORT

| DOCUMENTS CONSIDERED TO BE RELEVANT | | | CLASSIFICATION OF THE APPLICATION (Int. Cl. 3) |
|---|---|---|---|
| Category | Citation of document with indication, where appropriate, of relevant passages | Relevant to claim | |
| A | NATURE, vol. 192, no. 4808, 23th December 1961, F.P. DOYLE et al.: "New penicillins stable towards both acid and penicillinase", pages 1183-1184 * Complete article * -- | 1,7 | A 61 K 31/43<br>C 07 D 499/74<br>499/76 |
| A | NATURE, vol. 199, no. 4891, 27th July 1963, H.C. RICHARDS et al.: "Quinacillin: A new penicillin with unusual properties", pages 354-6 * Page 355, column 1, below, column 2 and page 356 * -- | 1,7 | TECHNICAL FIELDS SEARCHED (Int.Cl. 3)<br>A 61 K 31/43<br>C 07 D 499/74<br>499/76 |
| DA | THE JOURNAL OF ORGANIC CHEMISTRY, vol. 28, May-August 1963, D.A. JOHNSON et al.: "Penicillin sulfones", pages 1927-8 * Complete article * ---- | 1 | CATEGORY OF CITED DOCUMENTS<br>X: particularly relevant<br>A: technological background<br>O: non-written disclosure<br>P: intermediate document<br>T: theory or principle underlying the invention<br>E: conflicting application<br>D: document cited in the application<br>L: citation for other reasons<br>&: member of the same patent family, corresponding document |

✓ The present search report has been drawn up for all claims

| Place of search | Date of completion of the search | Examiner |
|---|---|---|
| The Hague | 07-07-1980 | CHOULY |

EPO Form 1503.1 06.78